# EUROPÄISCHE PATENTSCHRIFT

(11) **EP 1 255 538 B1**
(45) Veröffentlichungstag und Bekanntmachung des Hinweises auf die Patenterteilung: **22.10.2003**
(21) Anmeldenummer: 01913699.3
(22) Anmeldetag: 15.02.2001
(51) Int. Cl.: A61K 31/426, A61P 35/00

(54) **VERWENDUNG VON 2-METHYL-THIAZOLIDIN-2,4-DICARBONSÄURE UND / ODER IHRER PHYSIOLOGISCH VERTRÄGLICHEN SALZE ZUR HERSTELLUNG EINES MEDIKAMENTS ZUR BEHANDLUNG VON KREBSERKRANKUNGEN**
USE OF 2-METHYL-THIAZOLIDIN-2,4-DICARBOXYLIC ACID (2-MTDC) AND/OR PHYSIOLOGICALLY COMPATIBLE SALTS FOR THE MANUFACTURE OF A MEDICAMENT FOR TREATING AND/OR PREVENTING CANCERS
UTILISATION D'ACIDE 2-METHYL-THIAZOLIDIN-2,4-DICARBOXYLIQUE (2-MTDC) ET/OU DE SES SELS PHYSIOLOGIQUEMENT COMPATIBLES POUR LA PREPARATION D'UN MEDICAMENT POUR LE TRAITEMENT ET/OU LA PREVENTION DE CANCERS

(30) Priorität: 15.02.2000 DE 10008159
(43) Veröffentlichungstag der Anmeldung: 13.11.2002
(73) Patentinhaber: Susilo, Rudy, Dr., 50999 Köln (DE); Trommsdorff GmbH & Co.KG Arzneimittel, 52475 Alsdorf (DE)
(72) Erfinder: SUSILO, Rudy, 50999 Köln (DE); ROMMELSPACHER, Hans, 14193 Berlin (DE)
(74) Vertreter: Bucher, Ralf, Dipl.-Ing. (Univ.)
(86) Internationale Anmeldenummer: DE0100656
(87) Internationale Veröffentlichungsnummer: WO01060343

(56) Entgegenhaltungen:
- WO-A-98/38994
- L.B.WLODEK ET AL.: "Selective modulation of non-protein sulfhydryl levels in Ehrlich ascites tumor bearing mice" NEOPLASMA, Bd. 43, Nr. 4, 1996, Seiten 259-263, XP000974198 czech republic

## Beschreibung

Die Erfindung betrifft die Verwendung von 2-Methylthiazolidin-2,4-dicarbonsäure (2-MTDC) und/oder ihrer physiologisch verträglichen Salze zur Herstellung eines Medikaments zur Behandlung und/oder Prophylaxe von Krebserkrankungen.

In der Statistik der Todesursachen ist Krebs seit der Jahrhundertwende in den Industrieländern von der siebten auf die zweite Stelle vorgerückt. Trotz enormer Forschungsaufwendungen sterben in der Bundesrepublik Deutschland jährlich etwa 160.000 Menschen an Krebs. In den Vereinigten Staaten werden jährlich etwa 600.000 neue Krebsfälle diagnostiziert.

Die Abwehr von Krebserkrankungen ist zum einen durch persönliche Risikofaktoren eines Individuums und zum anderen durch die Möglichkeiten einer therapeutischen Intervention gekennzeichnet.

Viele der bekannten Antikrebsmittel besitzen starke Nebenwirkungen, wodurch bei der Krebsbehandlung gesunde Organe geschwächt und/oder geschädigt werden.

Somit besteht ein dringendes Bedürfnis, Substanzen für die Behandlung und Prophylaxe von Krebserkrankungen zur Verfügung zu stellen, welche keine starken Nebenwirkungen aufweisen.

Aufgabe der vorliegenden Erfindung ist es daher, eine physiologisch verträgliche Substanz zur Behandlung und/oder Prophylaxe von Krebserkrankungen zur Verfügung zu stellen.

Diese Aufgabe wird erfindungsgemäß durch die Verwendung von 2-Methyl-thiazolidin-2,4-dicarbonsäure (2-MTDC) und/oder ihrer physiologisch verträglichen Salze als physiologisch verträgliche Substanz zur Herstellung eines Medikaments zur Behandlung und/oder Prophylaxe von Krebserkrankungen gelöst.

Die Synthese von 2-Methyl-thiazolidin-2,4-dicarbonsäure (2-MTDC), ihre Verwendung als Hepatoprotektivum, sowie die Herstellung von 2-Methyl-thiazolidin-2,4-dicarbonsäure (2-MTDC) enthaltenden Arzneimitteln in Form von Dragees oder Salben sind aus DE-OS 21 16 629 bekannt. 2-MTDC ist für einige Anwendungen als Arzneimittel vorgeschlagen worden. So offenbart EP 989 16 811 die Verwendung von 2-Methyl-thiazolidin-2,4-dicarbonsäure (2-MTDC) als Mukolytikum und EP 989 16 809 beschreibt ein Kombinationspräparat aus 2-Methyl-thiazolidin-2,4-dicarbonsaure (2-MTDC) und Paracetamol.

Über die günstigen Einflüsse von 2-Methyl-thiazolidin-2,4-dicarbonsäure (2-MTDC) und ihrer physiologisch verträglichen Salze zur Prävention der Krebserkrankungen und als Therapeutikum bei Krebserkrankungen und/oder zur Reduktion der Nebenwirkungen von Cytostatika war bisher nichts bekannt.

Eine Beziehung zwischen der Verabreichung von 2-Methyl-thiazolidin-2,4-dicarbonsäure und der Glutathion-Konzentration in Leberzellen und in Tumorzellen von tumortragenden Mäusen beschreibt L. B. Wlodek et al. ("Selective modulation of non-protein sulfhydryl levels in Ehrlich ascites tumor bearing mice" NEOPLASMA, 1996, 43, 4, 259-263). Es wurde gezeigt, dass 2-MTDC die Konzentration von Glutathion in der Leber zu erhöhen vermag, wobei keine Erhöhung des Glutathionspiegels in den Tumorzellen nachgewiesen werden konnte. Daraus konnte die Schlussfolgerung gezogen werden, dass 2-MTDC durch selektive Erhöhung des Glutathionspiegels die Leber schützen kann, Tumorzellen jedoch nicht geschützt werden. Nicht untersucht wurde hingegen, ob die Verabreichung von 2-MTDC neben der protektiven Wirkung auf Leberzellen auch eine direkte Auswirkung auf das Tumorwachstum hat.

Überraschenderweise wurde gefunden, daß 2-Methylthiazolidin-2,4-dicarbonsäure (2-MTDC) und/oder ihre physiologisch verträglichen Salze zur Herstellung eines Medikaments zur Behandlung und/oder Prophylaxe von Krebserkrankungen wirkungsvoll eingesetzt werden können.

In Tierversuchen konnte belegt werden, daß 2-Methylthiazolidin-2,4-dicarbonsäure (2-MTDC) und/oder ihre physiologisch verträglichen Salze den Fortgang von Krebserkrankungen verlangsamt oder sogar aufhält.
Diese Studien wurden an mehreren tierexperimentellen Tumormodellen durchgeführt. Als Testsysteme galten Maus, Ratte und Hamster. Untersucht wurden unter anderem Hautpapillome, Brustadenokarzinome, Luftröhrenkrebs, Lungenadenokarzinome sowie Colonkarzinome und Blasenkrebs.

Die Wirkungsweise von 2-Methyl-thiazolidin-2,4-dicarbonsäure (2-MTDC) ist noch nicht genau bekannt. Es wird jedoch vermutet, daß 2-Methyl-thiazolidin-2,4-dicarbonsäure (2-MTDC) als "pro-drug" zur Freisetzng von L-Cystein dient, welches weiter zu Glutathion (γ-Glutamylcysteinylglycin, GSH) umgesetzt wird, dem die Funktion eines Radikalfängers zugeschrieben wird.

Daher besteht ein präventiver Ansatz bei Krebserkrankungen darin, daß mutagene Substanzen wie beispielsweise Nitrit- oder Nitrosoverbindungen mittels Radikalfänger abgefangen werden, bevor sie im Körper ihre schädigende Wirkung entfalten. Dies gelingt mit Antioxidantien wie körpereigenem Glutathion (GSH) oder synthetischem N-Acetylcystein (NAC).

Darmbakterien sind eine relevante Quelle für körpereigenes Glutathion (GSH). Bei einer Krebstherapie ist daher darauf zu achten, daß die körpereigenen Quellen für Glutathion oder andere Radikalfänger geschont und nicht beispielsweise durch eine Chemotherapie zusätzlich geschwächt werden, so daß keine zusätzliche Beeinträchtigung der Versorgung mit Glutathion (GSH) eintritt.

Glutathion (γ-Glutamylcysteinylglycin, GSH) ist die wichtigste natürliche Substanz, um die für die Körperzellen giftigen Sauerstoffradikale, die bei zahlreichen oxidativen Enzymreaktionen gebildet werden, abzufangen und zu beseitigen. Glutathion (GSH) kommt in jeder Körperzelle in hohen Konzentrationen vor, was verständlich ist, weil anderenfalls die entstandenen Sauerstoffradikale Bestandteile der Zellmembran zerstören würden, aber auch andere, intrazelluläre Vorgänge einschließlich Reparaturmechanismen im Bereich der Gene blockieren würden. Eine ungehinderte Wirkung der Sauerstoffradikale führt zum Zelluntergang.

Da reaktive Sauerstoffspezies (Sauerstoffradikale) die Karzinogenese beschleunigen, ist ein Abfangen dieser Moleküle ein weiterer Mechanismus, über den Glutathion (GSH) präventiv wirkt.

Eine entsprechende Wirkung wird auch dem N-Acetylcystein zugeschrieben. N-Acetylcystein enthält L-Cystein in maskierter Form, welches nach seiner Freisetzung im Körper in Glutathion (GSH) umgewandelt werden kann.

Zudem wurde eine weitere wichtige protektive Wirkung von Glutathion (GSH) und N-Acetylcystein (NAC) im Zusammenhang mit der Krebsentstehung beobachtet. Man fand, daß in Leberzellen die Zerstörung des genetischen Materials durch Bestrahlung und Karzinogene (z.B. 2AAF) bei gleichzeitiger Gabe von N-Acetylcystein (NAC) vermindert werden konnte.

Außer diesen protektiven Wirkungen zur Verhinderung der Entstehung von Krebs kann Glutathion (GSH) auch das Wachstum bereits entstandener Tumore vermindern. Auch N-Acetylcystein (NAC) war in mehreren tierexperimentellen Tumormodellen in diesem Sinne wirksam. Bei Patienten mit Lungen- oder Brustkrebs wurden in einem relativ frühen Stadium der Erkrankung deutlich verminderte Blutspiegel an L-Cystein und anderen Aminosäuren nachgewiesen (Zhang P.C., Pang C.P., Clin. Chem. 38, 1198-1199, 1992). Eine solche Verminderung wurde auch in C57BL/6-Mäusen mit Fibrosarkom gefunden (Hack V., Gross A., Kinscherf R., Bockstette M., Fiers W., Becke G. und Dröge W., FASEB J. 10, 1219-1226, 1996).

Über die Wirkungsmechanismen herrscht noch eine gewisse Unklarheit. In Bakterien wurde beobachtet, daß N-Acetylcystein (NAC) spontane und beispielsweise durch Bestrahlung induzierte Mutationen sowie die Bildung von Komplexen zwischen karzinogen wirkenden Substanzen und dem Erbmaterial (DNS) hemmt. In diesen Studien konnte auch eine Verzögerung der Entwicklung oder eine völlige Unterdrückung von Tumoren durch N-Acetylcystein (NAC) festgestellt werden.

Die Reduktion der unerwünschten Wirkungen von Cytostatika durch Gabe von thiolhaltigen Substanzen ist ein weiteres, vielversprechendes Anwendungsgebiet. Beispielsweise wurden Patienten mit Lungenkrebs mit Epirubicin behandelt. Bei gleichzeitiger Behandlung mit N-Acetylcystein (NAC) wurden die kardiotoxischen, unerwünschten Wirkungen von Epirubicin vermieden (Cipri A., Peverini M., Schiavo B. und Pozzar F., Eur. Respiration J. 7 (Suppl. 8) 391s 1994).

In einer Langzeitstudie wurde in Europa N-Acetylcystein (NAC) multizentrisch als präventives Medikament bei Risikopatienten für ein Lungenkarzinom getestet (EUROSCAN). Das Ergebnis war, daß N-Acetylcystein (NAC) zumindest in Forschungsstudien für diese Indikation vielversprechend ist (Van Zandwijk N., J. of Cellular Biochemistry 58, Suppl. 22, 24-32, 1995).

Nach dem derzeitigen Kenntnisstand ist die Wirkungsweise von 2-Methyl-thiazolidin-2,4-dicarbonsäure (2-MTDC) noch nicht vollständig aufgeklärt. Es kann jedoch eine dem N-Acetylcystein (NAC) äquivalente Wirkungsweise angenommen werden, welche zu dem Schlüsselmolekül Glutathion (GSH) führt. Dieses ist in der Lage, Radikale und vorrangig Sauerstoffradikale unschädlich zu machen.

Geht man von diesem recht unvollkommenen und noch viele Fragen aufwerfenden Wirkmechanismus aus, so sollte als vorrangiges Forschungsziel die Bereitstellung und Verwendung von Substanzen gefördert werden, welche im Körper in kontrollierter Weise und ohne Auftreten toxischer Produkte L-Cystein in benötigter Menge und am rechten Ort freisetzen.

Die bekannten und bisher angewandten Verbindungen des Standes der Technik erfüllen diese Aufgabe nur unzureichend.

Um diesen therapeutischen Ansatz zu realisieren, läge es nahe, die natürliche Substanz Glutathion (GSH) zu applizieren. Glutathion kommt jedoch selbst nicht in Frage, weil es zum einen im Magen zerstört würde und zum anderen nicht in die Zellen hineintransportiert werden kann. Die Zellen verfügen über keinen entsprechenden Transportmechanismus.

Eine direkte Applikation von L-Cystein verbietet sich ebenfalls, weil L-Cystein giftig ist, wie in Zellkulturen und am Beispiel neugeborener Mäuse und Ratten gezeigt werden konnte. Die Applikation des giftigen L-Cysteins führt zum Absterben von Hirnzellen (Karlsen R.L., Grofova Y., Malthe-Sorensen D. und Farnum E., Exp. Brain. Res. 208, 167-180, 1981). Eine Möglichkeit, diese Toxizität zu umgehen, ist die Applikation einer sogenannten "pro-drug", also eines Vorläufermedikaments, aus dem die wirksame Aminosäure erst im Körper kontrolliert freigesetzt wird.

Die Substanzen Glutathion (GSH) oder L-Cystein müssen also durch Vorstufen substituiert werden, die im Körper in L-Cystein umgewandelt werden können, das dann für die Synthese von Glutathion zur Verfügung steht. Die bekannteste Vorstufe ist N-Acetylcystein (NAC).

Die Freisetzung von L-Cystein aus N-Acetylcystein (NAC) erfolgt nur zu einem geringen Teil durch Hydrolyse. Vorwiegend erfolgt die Freisetzung durch eine Aminosäuren-N-Deacylase, die beispielsweise im Cytosol von Leberzellen nachgewiesen werden konnte (Wlodek, L., Rommelspacher, H., Susilo, R., Radomski, J. und Hoefle, G., Biochem. Pharmacol. 46, 917-928, 1993).

Im allgemeinen wird davon ausgegangen, daß es sich bei N-Acetylcystein (NAC) um ein Medikament geringer Toxizität handelt. Allerdings gibt es einige, jedoch wenig beachtete Berichte, welche belegen, daß das Toxizitätsrisiko von N-Acetylcystein (NAC) unterschätzt wird (Estrela J.M., Saez G.T., Such L. und Vina J., Biochem. Pharmacol. 32, 3483-3485, 1983 und Vina J., Romero F.J., Saez G.T., Pallardo F.V., Experimentia 39, 164-165, 1983).

Aufgrund des beschriebenen Risikos toxischer Reaktionen, ist es unerläßlich, nach Alternativen zu N-Acetylcystein (NAC) Ausschau zu halten.

Eine Alternative zu N-Acetylcystein (NAC) könnten Thiazolidine sein, welche ein Vorstufenmedikament zu Glutathion (GSH) darstellen.

Die Kondensation von Carbonylgruppen enthaltenden Stoffen mit L-Cystein zu Thiazolidinen wurde bereits beschrieben (Susilo R., Rommelspacher H. und Hoefle G., Journal of Neurochem. 52, 1793-1800, 1989). Wichtig ist dabei, daß die Thiazolidine ein Reservoir für L-Cystein bilden, aus dem bei Bedarf die Aminosäure freigesetzt werden kann.

Ein strukturell einfaches Thiazolidin ist das Kondensationsprodukt zwischen Formaldehyd und L-Cystein. Metaboliten dieser Substanz stellten sich allerdings als neurotoxisch heraus.

Das Kondensationsprodukt zwischen Acetaldehyd und L-Cystein ist als ein Vorläufermedikament ebenfalls ungeeignet, da es unter physiologischen Bedingungen spontan in seine Komponenten zerfällt (Wlodek L., Rommelspacher H., Susilo R., Radomski J. und Hoefle G., Biochem. Pharmacol. 46, 917-928, 1993).

Da die beschriebenen Thiazolidine den pharmakologischen Anforderungen nicht gerecht werden, bedarf es der Suche nach pharmakologisch einsetzbaren Thiazolidinderivaten.

Es wurde nun gefunden, daß das Kondensationsprodukt aus Brenztraubensäure und L-Cystein die aufgestellten Anforderungen am besten erfüllt.
Bei der Freisetzung von L-Cystein aus 2-Methyl-thiazolidin-2,4-dicarbonsäure (2-MTDC) entsteht als Nebenprodukt physiologisch unbedenkliches Pyruvat. Pyruvat ist eine physiologische Substanz, die im Körper in den freigesetzten Mengen völlig unschädlich ist. 2-Methyl-thiazolidin-2,4-dicarbonsäure (2-MTDC) zeichnet sich deshalb im Gegensatz zu N-Acetylcystein (NAC) durch eine sehr gute Verträglichkeit aus. Es gibt sogar Hinweise auf eine protektive Wirkung von Pyruvat (Rastellini C., Cicalese L., Zeevi A., Matten C., Stauko R.T., Starzl T.E. und Rao A.S., Transplantation Proceedings 27, 3383-3384, 1995). Pyruvat entsteht unter physiologischen Bedingungen aus Glucose und wird im Zitronensäurezyklus zur Energiegewinnung der Zelle benötigt.

Überraschenderweise wurde festgestellt, daß die Behandlung mit 2-Methyl-thiazolidin-2,4-dicarbonsäure (2-MTDC) nicht nur die Zerstörung des zellulären Abwehrsystems verhindert, wie am Beispiel von Leberzellen gezeigt werden konnte (Wlodek L. und Rommelspacher H., Alcohol and Alcoholism 29, 649-657, 1994), sondern daß auch ein lang anhaltender Effekt dieser schützenden Wirkung von 2-Methyl-thia-zolidin-2,4-dicarbonsäure (2-MTDC) erzielt wird.

Die Freisetzung von L-Cystein und die Bildung von Glutathion (GSH) war noch nach 12 Stunden im Lebergewebe von Mäusen nachweisbar (2-MTDC 1,2 mmol/kg, intraperitoneal appliziert), führte nach 12 Stunden zu einer Erhöhung des GSH-Spiegels auf 112,0% (P<0,01). Die intraperitoneale Applikation von 2,4 mmol 2-MTDC pro kg Körpernaßgewicht führte zu einer Erhöhung des GSH-Spiegels in der Leber auf 154,5% der Referenzwerte, P<0,001. Die Versuchsergebnisse sind in Tabelle 1 zusammengestellt.

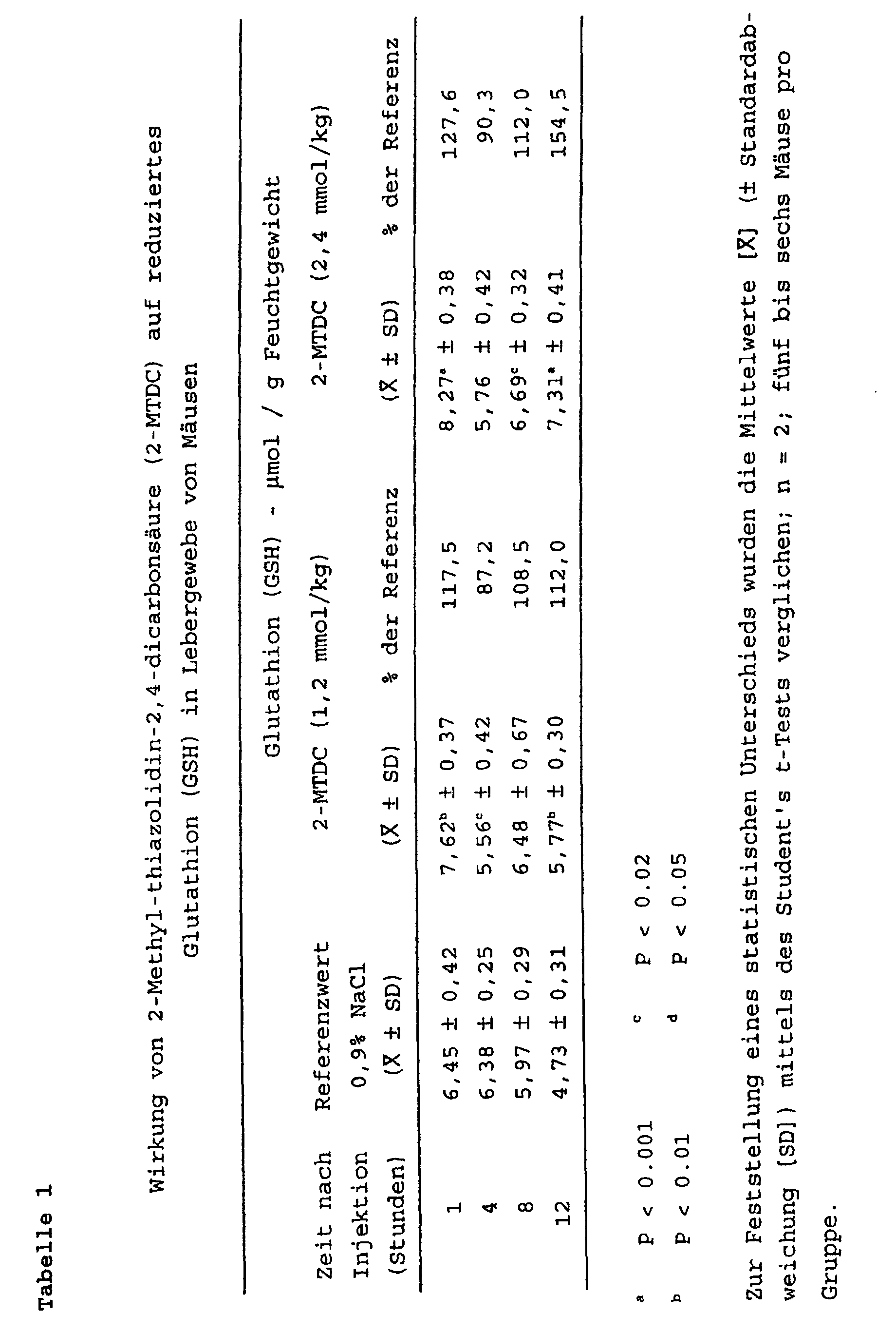

Diese Beobachtungen sprechen für eine enzymatisch regulierte Freisetzung von L-Cystein aus 2-Methyl-thiazolidin-2,4-dicarbonsäure (2-MTDC). Dies führt zu einer dem Bedarf des Körpers angepaßten, dosisabhängigen Freisetzung von L-Cystein. Dadurch werden sehr hohe Konzentrationen des giftigen L-Cysteins vermieden, wie sie beispielsweise nach einer Dauertherapie mit N-Acetylcystein (NAC) beobachtet werden. Dies trägt ebenfalls zu einer besseren Verträglichkeit von 2-Methyl-thiazolidin-2,4-dicarbonsäure (2-MTDC) bei.

Aufgrund der wahrscheinlich enzymatisch regulierten Freisetzung von L-Cystein aus 2-Methyl-thiazolidin-2,4-dicarbonsäure (2-MTDC) unter Abspaltung des physiologisch unbedenklichen Pyruvats zur kontrollierten dosisabhängigen Nachlieferung von vom Organismus benötigtem L-Cystein, ist 2-Methyl-thiazolidin-2,4-dicarbonsäure (2-MTDC) den bisher bekannten Verbindungen, wie N-Acetylcystein (NAC), deutlich überlegen.

Die vom N-Acetylcystein (NAC) bekannten toxischen Nebenwirkungen können durch die Verwendung von 2-Methylthiazolidin-2,4-dicarbonsäure (2-MTDC) deutlich verringert bzw. sogar vermieden werden.

Legt man die bisher erforschte Wirkungsweise von N-Acetylcystein (NAC) zugrunde, so ist verständlich, daß auch 2-Methyl-thiazolidin-2,4-dicarbonsäure (2-MTDC) eine Reduktion der Bildung der freien Radikale und eine Erhöhung der Sulfhydrylgruppen-Konzentration im Organismus hervorruft. Dies führt zu einer cytoprotektiven Wirkung dieser Verbindung.

2-Methyl-thiazolidin-2,4-dicarbonsäure (2-MTDC) führt daher zu einer Reduktion der Nebenwirkungen von Cytostatika durch Minderung oder Beseitigung des Thiolgruppendefizits, welches sich in einem Mangel an L-Cystein und/oder Glutathion (GSH) äußert.

Der Einsatz von 2-Methyl-thiazolidin-2,4-dicarbonsäure (2-MTDC) zur Prävention und gegen Progression der Krebserkrankungen sowie zur Reduktion der unerwünschten Wirkungen bei einer Cytostatikatherapie ist daher medizinisch und wissenschaftlich sinnvoll und empfehlenswert.

## Patentansprüche

1. Verwendung von 2-Methyl-thiazolidin-2,4-dicarbonsäure und/oder ihrer physiologisch verträglichen Salze zur Herstellung eines Medikaments zur Behandlung und/oder Prophylaxe von Krebserkrankungen.

2. Verwendung von 2-Methyl-thiazolidin-2,4-dicarbonsäure und/oder ihrer physiologisch verträglichen Salze nach Anspruch 1 zur Herstellung eines Medikaments zur Behandlung von Krebserkrankungen innerer Organe.

3. Verwendung von 2-Methyl-thiazolidin-2,4-dicarbonsäure und/oder ihrer physiologisch verträglichen Salze nach Anspruch 1 zur Herstellung eines Medikaments zur Behandlung von Lungen-, Magen-, Darm-, Pankreas-, Nieren- und Leberkrebs.

4. Verwendung von 2-Methyl-thiazoiidin-2,4-dicarbonsäure und/oder ihrer physiologisch verträglichen Salze nach Anspruch 1 zur Herstellung eines Medikaments zur Behandlung von Brustkrebs, Hautkrebs, Blasenkrebs, Speiseröhren- und Luftröhrenkrebs.

5. Verwendung von 2-Methyl-thiazolidin-2,4-dicarbonsäure und/oder ihrer physiologisch verträglichen Salze nach Anspruch 1 zur Herstellung eines Medikaments zur Vermeidung von unerwünschten Arzneimittelwirkungen der Cytostatika.

## Claims

1. Use of 2-methyl-thiazolidine-2,4-dicarboxylic acid and/or its physiologically tolerable salts for the manufacture of a medicament for treatment and/or prophylaxis of cancers.

2. The use of 2-methyl-thiazolidine-2,4-dicarboxylic acid and/or its physiologically tolerable salts according to claim 1 for the manufacture of a medicament for treatment of cancers of internal organs.

3. The use of 2-methyl-thiazolidine-2,4-dicarboxylic acid and/or its physiologically tolerable salts according to claim 1 for the manufacture of a medicament for treatment of pulmonary, gastric, pancreatic, renal and hepatic carcinomas.

4. The use of 2-methyl-thiazolidine-2,4-dicarboxylic acid and/or its physiologically tolerable salts according to claim 1 for the manufacture of a medicament for treatment of mammary, skin, bladder, esophageal and tracheal cancer.

5. The use of 2-methyl-thlazolidine-2,4-dicarboxylic acid and/or its physiologically tolerable salts according to claim 1 for the manufacture of a medicament for prevention of undesirable side effects of cytostatics.

## Revendications

1. Utilisation d'acide 2-méthylthiazolidine-2,4-dicarboxylique et/ou de ses sels physiologiquement acceptables pour la préparation d'un médicament pour le traitement et/ou la prophylaxie de maladies cancéreuses.

2. Utilisation d'acide 2-méthylthiazolidine-2,4-dicarboxylique et/ou de ses sels physiologiquement acceptables selon la revendication 1, pour la préparation d'un médicament pour le traitement de maladies cancéreuses d'organes internes.

3. Utilisation d'acide 2-méthylthiazolidine-2,4-dicarboxylique et/ou de ses sels physiologiquement acceptables selon la revendication 1, pour la préparation d'un médicament pour le traitement du cancer des poumons, de l'estomac, des intestins, du pancréas, des reins et du foie.

4. Utilisation d'acide 2-méthylthiazolidine-2,4-dicarboxylique et/ou de ses sels physiologiquement acceptables selon la revendication 1, pour la préparation d'un médicament pour le traitement du cancer du sein, du cancer de la peau, du cancer de la vessie, du cancer de l'oesophage et de la trachée.

5. Utilisation d'acide 2-méthylthiazolidine-2,4-dicarboxylique et/ou de ses sels physiologiquement acceptables selon la revendication 1, pour la préparation d'un médicament pour éviter les effets non désirés des médicaments cytostatiques.
